# EUROPEAN PATENT SPECIFICATION

(11) **EP 2 330 208 B1**
(45) Date of publication and mention of the grant of the patent: **18.10.2017**
(21) Application number: 10181903.5
(22) Date of filing: 24.05.2005
(51) Int. Cl.: A61K 49/18, A61K 49/00, A61K 51/12, C12N 15/88, A61K 47/69, G01N 33/58

(54) **Nanoparticles comprising RNA ligands**
Nanopartikel enthaltend RNA-Liganden
Nanoparticules portant des ligands ARN

(30) Priority: 24.05.2004 US 573805 P; 24.05.2004 GB 0411537
(43) Date of publication of application: 08.06.2011
(62) Divisional of application: 05746540.3
(73) Proprietor: Midatech Ltd., Abingdon, Oxford Oxfordshire 0X13 6BH (GB)
(72) Inventor: Rademacher, Thomas William, Oxford, Oxfordshire OX13 6BH (GB); Gumaa, Khalid, Oxford, Oxfordshire OX13 6BH (GB); Martin-Lomas, Manuel, 20009 San Sebastián, Guipúzcoa (ES); Penades, Soledad, 20009 San Sebastián (ES); Ojeda, Rafael, 41010 Seville (ES); Barrientos, Africa, 48600 Sopelana (Vizcaya) (ES)
(74) Representative: Kiddle, Simon John

(56) References cited:
- EP-A- 1 249 502
- EP-A1- 0 990 903
- WO-A-98/04740
- WO-A-2004/108165
- WO-A1-2006/016978
- WO-A2-01/51665
- WO-A2-02/31191
- WO-A2-03/099227
- WO-A2-2005/001143
- WO-A2-2006/078289
- US-B1- 6 361 944
- DUBERTRET B ET AL: "Single-mismatch detection using gold-quenched fluorescent oligonucleotides", NATURE BIOTECHNOLOGY, vol. 19, no. 4, April 2001 (2001-04), pages 365-370, XP002224627, ISSN: 1087-0156
- JIN R: "What controls the melting properties of DNA-linked gold nanoparticle assemblies?", JOURNAL OF THE AMERICAN CHEMICAL SOCIETY, AMERICAN CHEMICAL SOCIETY, US, vol. 125, 15 January 2003 (2003-01-15), pages 1643-1654, XP002294467, ISSN: 0002-7863, DOI: DOI:10.1021/JA021096V

## Description

### Field of the Invention

The present invention relates to nanoparticles, and more particularly to nanoparticles comprising RNA ligands such as small interfering RNA (siRNA) and micro RNA (miRNA), and their use in a range of applications.

### Background of the Invention

Small RNA molecules have been found to play multiple roles in regulating gene expression. These include targeted degradation of mRNAs by small interfering RNAs (siRNAs), post transcriptional gene silencing (PTGs), developmentally regulated sequence-specific translational repression of mRNA by micro-RNAs (miRNAs) and targeted transcriptional gene silencing. RNAi activity limits transposon mobilization and provides an antiviral defence (Pal-Bhadra et al, 2004). A role for the RNAi machinery and small RNAs in targeting of heterochromatin complexes and epigenetic gene silencing at specific chromosomal loci has also been demonstrated (Verdel et al, 2004). Double-stranded RNA (dsRNA)-dependent post transcriptional silencing, also known as small inhibitory RNA (siRNA) or RNA interference (RNAi), is a phenomenon in which dsRNA complexes can target specific genes of homology for silencing in a short period of time. It acts as a signal to promote degradation of mRNA with sequence identity. A 20-nt siRNA is generally long enough to induce gene-specific silencing, but short enough to evade host response (Elbashir et al, 2001).

The decrease in expression of targeted gene products can be extensive with 90% silencing induced by a few molecules of siRNA.

Since the delivery of small-molecule oligonucleotides can bypass the difficulties associated with gene therapy, the use of siRNA may have advantages over traditional gene therapy. To date, efficient delivery of vector-based therapeutic genes *in vivo* remains an obstacle to successful gene therapy. It has been observed that although knockdown of the target gene by siRNA is not permanent, a single siRNA transfection can lead to a prolonged inhibition of the target protein in the parent as well as progeny cells (Tuschl, 2001). However, there is still a problem in the art in delivery of siRNA.

EP 1 249 502 A describes semiconductor nanoparticle beads having DNA covalently bonded to the surface of the beads.

WO 98/04740 A describes nanoparticles having oligonucleotides attached to them and uses of these in assays for detecting nucleic acids and separating the selected nucleic acid from other nucleic acids.

EP 0 990 903 A1 describes fluorescent semiconductor nanocrystals associated to a compound and their use in biological applications.

WO 01/51665 A2 describes nanoparticles having oligonucleotides attached to them and uses of these in assays for detecting nucleic acids and separating the selected nucleic acid from other nucleic acids.

Dubertret et al., 2001, NATURE BIOTECHNOLOGY, Vol. 19, No. 4, pp. 365-370, describes a hybrid material composed of a single-stranded DNA (ssDNA) molecule, a 1.4 nm diameter gold nanoparticle, and a fluorophore that is highly quenched by the nanoparticle through a distance-dependent process.

US 6,361,944 B1 describes methods of detecting a nucleic acid, comprising contacting the nucleic acid with one or more types of particles having oligonucleotides attached thereto.

WO 03/099227 describes compositions and methods relating to siRNA polynucleotides that interfere with the expression of PTP1B.

WO 02/31191 describes a dendritically amplified detection method and system for the detection of a target nucleic acid in a sample solution.

Jin et al., 2003, J. AM. CHEM. SOC., Vol. 125, pp. 1643-1654, describes the relative importance of nanoparticle, oligonucleotide, and environmental variables that contribute to the observed sharp melting transitions associated with DNA-linked nanoparticle structures.

WO 2004/108165 describes magnetic nanoparticles employed as substrate for immobilising a plurality of ligands. Also disclosed are uses of these magnetic nanoparticles as therapeutic and diagnostic reagents, and in the study of ligand-mediated interactions.

WO 2005/001143 describes a method and kit for label-free detection of global gene expression using nanoparticles probes in an array assay format.

WO 2006/078289 describes screening methods, compositions and kits for detecting the presence or absence of one or more target analytes in a sample. Reporter oligonucleotides are utilized as biochemical barcodes for detecting multiple protein structures.

WO 2006/016978 describes compositions and methods involving analog nucleic acids, such as PNA and L-DNA for the detection of nucleic acids.

WO 02/32404 (Consejo Superior de Investigaciones Scientificas) discloses nanoparticles formed from metal or semiconductor atoms in which ligands comprising carbohydrates are covalently linked to the core of the nanoparticles. These nanoparticles are used for modulating carbohydrate mediated interactions and are soluble and non-toxic. PCT application claiming priority from GB-A-0313259.4 (Consejo Superior de Investigaciones Scientificas and Midatech Limited) discloses magnetic nanoparticles having cores comprising passive and magnetic metal atoms, the core being covalently linked to ligands.

### Summary of the Invention

Broadly, the present invention relates to nanoparticles comprise having a core including metal and/or semiconductor atoms, the core being linked to RNA ligands. The RNA ligands are typically short RNA sequences designed to mimic small interfering RNA (siRNA) and micro-RNA sequences (miRNA). The nanoparticles can be used to deliver the RNA ligands and have applications in a wide range of applications, in *in vitro* systems and for therapeutic or diagnostic applications. By way of example, the nanoparticles of the present invention may be employed (1) for targeted transcriptional gene silencing, (2) for targeted mRNA degradation, (3) for imaging mRNA, (4) for inhibiting pathways by employing a plurality of RNA ligands on the same or different nanoparticles, (5) for aerosol delivery, e.g. to the lungs, (6) in combination with mRNA silencing for targeting siRNA resistant mRNA and (7) for use a tool in functional genomics.

In the art, short RNA sequences are termed "short interfering RNAs" (siRNAs) or "microRNAs" (miRNAs) depending in their origin. Both types of sequence may be used to down-regulate gene expression by binding to complimentary RNAs (nmRNA) and either triggering mRNA elimination (RNAi) or arresting mRNA translation into protein. siRNA are derived by processing of long double stranded RNAs and when found in nature are typically of exogenous origin. Micro-interfering RNAs (miRNA) are endogenously encoded small non-coding RNAs, derived by processing of short hairpins. Both siRNA and miRNA can inhibit the translation of mRNAs bearing partially complimentary target sequences without RNA cleavage and degrade mRNAs bearing fully complementary sequences. The RNAi pathway also acts on the genome as discussed in Science, 301: 1060-1061, 2003).

The RNA associated with the nanoparticles may be single stranded or double stranded (duplex). Where miRNA-like sequences are used as ligands, the RNA sequences may be hairpins, that is include partially complementary regions towards their ends that can anneal to form the hairpin. The nanoparticles may optionally comprise further types of ligands, such as carbohydrates to form glyconanoparticles, and/or more than one species of siRNA. The nanoparticles and their uses are discussed in more detail below. Advantageously, the attachment of the siRNA to the nanoparticle may provide protection for the siRNA from exoribonucleases present in the blood, tissue culture media or within cells.

Accordingly, in a first aspect, the present invention provides a method for detecting and/or imaging mRNA employing nanoparticles which comprise a core including metal and/or semiconductor atoms, wherein the core is covalently linked to a plurality of ligands which comprise:
(i) at least one RNA ligand comprising (a) a siRNA or miRNA molecule between 17 and 30 ribonucleotides in length; and
(ii) a ligand comprising a carbohydrate group,
wherein the method comprises contacting the nanoparticles with a sample or cells containing target mRNA under conditions in which the RNA ligands present on the nanoparticles are capable of interacting with target mRNA and detecting the nanoparticle-RNA-mRNA complex, and wherein the core of the nanoparticle has a mean diameter between 0.5 and 10 nm.

In some cases, the step of detecting the complex uses an inherent property of the nanoparticles or is done by detecting a label associated with the nanoparticle.

In particular, the label may be a magnetic label, a quantum dot or a radionuclide.

In some cases, the method is carried out *in vitro* on a sample containing the mRNA.

In some cases, the method is for detecting mRNA in cells.

In some cases, the method is a tool for functional genomics.

In some cases, the nanoparticle is covalently linked to the RNA ligand via a linker group. In particular, the linker group may be a thiol group, an ethylene group or a peptide group.

In some cases, the ligand is a siRNA ligand and comprises a 3' overhang of 2 ribonucleotides.

In some cases, a first sense or antisense strand of a RNA molecule is covalently linked to a nanoparticle core via its 5' and/or 3' end. In particular, a second strand of the RNA molecule which is complementary to the first strand may be annealed to the first strand of the RNA molecule. In certain cases, the second RNA strand may be covalently linked to a nanoparticle core via its 5' and/or 3' end.

In some cases, the first and second strands of the RNA molecule are separately linked to nanoparticle cores and subsequently annealed together.

In some cases, the core of the nanoparticle comprises gold atoms.

Embodiments of the present invention will now be described by way of example and not limitation with reference to the accompanying figures.

### Brief Description of the Figures

**Figure 1** shows a transmission electron micrograph of RNA-Au-Glc nanoparticles.
**Figure 2** shows the testing for the presence of RNA in the prepared nanoparticles. (a) Without UV light: 1. RNA-Au-Glc nanoparticles + EtBr; 2. Glc-Au + EtBr; 3. Glc-Au; 4. residue of washing solution+ EtBr. (b) With UV light: 1. RNA-Au-Glc nanoparticles + EtBr; 2. Glc-Au + EtBr; 3. Glc-Au; 4. residue of washing solution + EtBr.
**Figure 3a** shows a Western blot of Her-2/neu protein from equal volumes of lysates of SKBR3 cells 48 hours after transfection with a Her-2/neu siRNA. C = Control (untreated) cells; Au = Cells treated with siRNA coupled to gold nanoparticles without RNAiFectamine; S = Silencing siRNA with RNAiFectamine; NS = Non-silencing siRNA with RNAiFectamine.
**Figure 3b** shows a Western blot of Her-2/neu protein from equal volumes of OVCAR cell lysates 72 hours after transfection with a Her-2/neu siRNA. C = Control (untreated) cells; Au = Cells treated with siRNA coupled to gold nanoparticles without RNAiFectamine.
**Figure 4** shows a schematic representation of a preferred nanoparticle of the invention comprising siRNA and carbohydrate ligands.
**Figure 5** shows the effect on cell proliferation on OVCAR cells transfected with siRNA alone (**A**) or with siRNA-nanoparticles (**B**) at 0.25µg (diamonds), 0.5µg (squares), 1.0µg (triangles), 1.5µg (grey cross) and 2.0µg (black cross) siRNA-nanoparticles per 1000 cells. X axis = days; Y axis = cell number (log₁₀)
**Figure 6** shows the effect on cell proliferation on OVCAR cells transfected with siRNA-nanoparticles with and without transfection reagent. Three concentrations of nanoparticles were used:
   1: with transfection reagent (squares) and without transfection reagent (diamonds);
   2: with transfection reagent (grey crosses) and without transfection reagent (triangles);
   3: with transfection reagent (circles) and without transfection reagent (black crosses).
   X axis = days; Y axis = cell number (log₁₀)

### Detailed Description

### Examples

The Her-2/neu oncogene and its encoded product p185Her-2/neu belong to the epidermal growth factor receptor tyrosine kinases (Bargmann et al, 1986). The HER receptor family consists of four transmembrane tyrosine kinases: EGFR (also known as Her-1 or erbB-1), erbB-2 (Her-2), erbB-3 (Her-3), and erbB-4 (Her-4). Her-2/neu signalling pathways are known to play critical roles in cell growth and differentiation, malignant transformation, and resistance to chemotherapeutic agents (Yarden & Sliwkowski, 2001). Her-2/neu is over-expressed in about one third of cases of human breast or ovarian cancers, and its over-expression is associated with poor prognosis (Berchuck et al, 1990).

Numerous attempts have been made to inhibit Her-2/neu expression in cancer cells as a potential therapeutic approach. A humanized monoclonal antibody against Her-2/neu (Trastuzumab or Herceptin) has been effective in Her-2/neu-overexpressing metastatic cancer (Mendelsohn & Baselga, 2000; Baselga et al, 1996) but was found to up-regulate Her-3 expression. An antisense oligonucleotide against Her-2/neu has been shown to induce apoptosis in human breast cancer cell lines that overexpress Her-2/neu (Roh et al, 2000). Gene therapy with E1A, delivered by liposomes or by adenoviral vectors, can reduce mortality among tumour-bearing mice in a model of Her-2/neu-overexpressing ovarian cancer and can reduce the incidence of distant metastases in a model of breast cancer (Chang et al, 1996).

The down regulation of Her-2/neu expression was found to lead to decreases in PI3K, Akt, and phosphorylated Akt which resulted in decreased expression of cyclin D1, a cyclin involved in the regulation of G0/G1 cell arrest and oncogenic transformation (Sherr & Roberts, 1999). A recent study comparing the efficacy of antisense oligonucleotides and siRNA demonstrated that siRNAs are at least 10 times more efficient on a nM basis at silencing a reporter gene (Miyagishi et al, 2003). Several previous studies have demonstrated that Her-2/neu stimulates the transcription of VEGF, a potent proangiogenic factor (Kumar & Yarmand-Bagheri, 2001) the level of which was markedly decreased after silencing of Her-2/neu expression. Down regulation of Her-2/neu by retroviral siRNA increased thrombospondin-1 levels, a powerful inhibitor of angiogenesis (Izumi et al, 2002). *In vitro* data demonstrated that HER2 siRNA treatment also significantly up-regulates HLA class I surface expression in human tumours (Choudhury et al, 2004).

### a. Strategy: Silencing

Her2/Neu cDNA Target Sequence: **AAG CCT CAC AGA GAT CTT GAA**
a) Sense: **5'- G CCU CAC AGA GAU CUU GAAdTdT** - **3'**
b) Antisense: **3'** - **dTdTC GGA GUG UCU CUA GAA CUU** - **5'**
c) Sense: **5'- G CCU CAC AGA GAU CUU GAAdTdT** - **3**'**SS**
d) Antisense:**3**'**SS**-**dTdTC GGA GUG UCU CUA GAA CUU** - **5'**

### b. Annealing

Silencing:

5′ (a) 3′
3′ (b) 5′

5′ (a) 3′
3′SS (d) 5′

5′ (c) 3′SS
3′ (b) 5′

5′ (c) 3′SS
3SS′ (d) 5′

### c. Possible GNP combinations

| **siRNA** | **Anneal** | **GNP** | | |
|---|---|---|---|---|
| | | **Glc**- | **Glcβ4GlcNAc** | **Glcβ4GlcNH₂** |
| **Silencing** | a-b | | | |
| | a-d | | | |
| | c-b | X | | 30 |
| | c-d | | | |

| | | | | |
|---|---|---|---|---|
| X = used in this study. | | | | |

### d. Methods

### 1. Cell Lines

SK-BR-3 Human Mammary adenocarcinoma from ATCC (Cat.# HTB-30). OVCAR-3 Human ascites adenocarcinoma from NCI-Frederick Cancer DCTD Tumor/cell line repository (vial 0502296).

### 2. siRNA Stock Solutions

The Her-2/neu DNA target Sequence chosen was AAGCCTCACA GAGATCTTGAA.

The sense siRNA had a sequence r(GCCUCACAGAGAUCUUGAA) d(TT)_{3ThSS}. (MW of K-salt 7416.25) and the antisense sequence r(UUCAAGAUCUCUGUGAGGC) d(TT)_{3ThSS} (MW of K-salt 7409.57) were obtained from Qiagen.

The control (non-silencing) siRNA duplex sequences from Qiagen (Cat# 1022076) where sense r(UUC UCC GAA CGU GUC ACG U)d(TT) and antisense r(ACG UGA CAC GUU CGG AGA A)d(TT) and the MW of the annealed K-salt was 14839.5.

Dissolve contents (296.65 µg) of one sense siRNA tube in 1 ml sterile buffer (100 mM potassium acetate, 30 mM Hepes-KOH, 2 mM magnesium acetate pH 7.4) to make a 40 µM stock. Each µl will contain 0.297 µg siRNA.

The contents (296.38 µg) of one antisense siRNA tube were dissolved in 1 ml sterile buffer (100 mM potassium acetate, 30 mM Hepes-KOH, 2 mM magnesium acetate pH 7.4) to make a 40 µM stock. Each µl contained 0.296 µg siRNA.

To anneal, 30 µl of each RNA oligo solution was combined with 15 µl of 5X annealing buffer. The final buffer concentration was 50 mM Tris, pH 7.5 - 8.0, 100 mM NaCl in DEPC-treated water. The final volume was 75 µl and the final concentration of siRNA duplex was 16 µM.

The solution was incubated for 1 minute in a water bath at 90-95°C, and allowed to cool to room temperature (i.e. below 30°C). The tube was centrifuged briefly to collect all liquid at the bottom of the tube. Slow cooling to room temperature took 45-60 minutes. The resultant solution was stored at -20°C until ready to use and was resistant to repeated freezing and thawing.

### 3. siRNA Nanogold Stock Solution

### General Methods

HAuCl₄ (99.999%) and NaBH₄ were purchased from Aldrich Chemical Company. 2-thioethyl-β-D-glucopyranoside was synthesized in our laboratory using standard procedures. For all experiments and solutions, Nanopure water (18.1 mΩ) treated with DEPC (diethylpirocarbonate) was used. All eppendorfs, spatulas and vials were RNase free. Annealed double-stranded siRNA was purchased from Qiagen-Xeragon Inc. The specifications were:
DNA target sequence AAGCCTCACAGAGATCTTGAA.

Sense siRNA r (GCCUCACAGAGACUUGAA) d (TT) 3'-Thiol-(SS)-C3-linker on 3'
(MW of K-salt 7416.25)
Antisense r(UUCAAGAUCUCUGUGAGGC)d(TT)
(MW of K-salt 7409.57)

### e. Preparation of RNA-Au-Glc nanoparticles

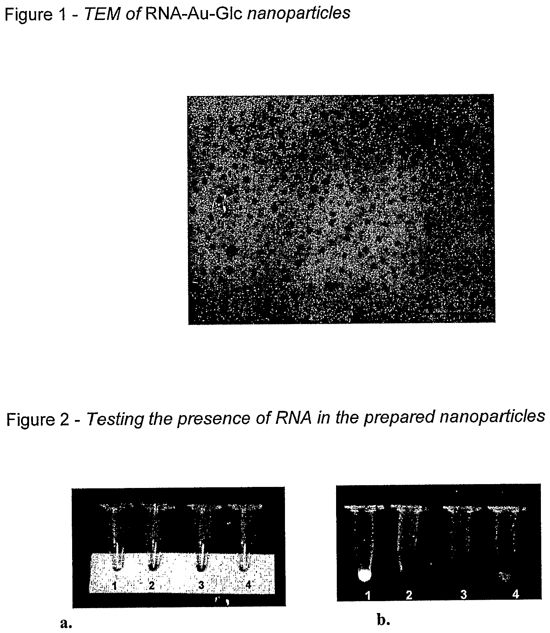

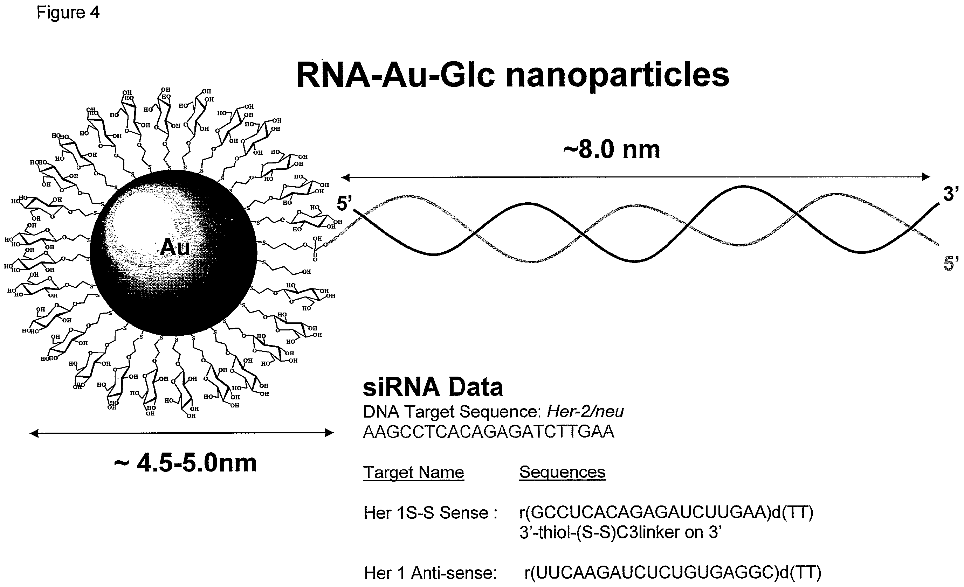

To a solution of 2-thioethyl-β-D-glucopyranoside (0.9 mg, 3.75 µmol) and siRNA (0.148 mg, 0.01 µmol) in TRIS buffer 100 mM, pH 7.7 (250 µL), an aqueous HAuCl₄ solution (22 µL, 0.025M) was added. Then, IN aqueous solution of NaBH₄ (30 µL) was added in several portions with rapid shaking. The brown suspension formed was shaken for an additional 1h at 4°C. The suspension was purified by centrifugal filtering (AMICON MW 10000, 30min, 4°C, 14000 rpm). The process was repeated twice, washing with 125 µL of TRIS buffer. The residue in the AMICON filter was dissolved in 250 µL of TRIS buffer and lyophilised to afford 4 mg of RNA-Au-Glc nanoparticles (resuspension of the solid in 1mL of water should give a 6±1µM solution of RNA in 20 mM TRIS buffer). The filtrate was desalted using AMICON (MW 3000, 4°C, 14000 rpm) and lyophilised. The weight of the residue was < 50 µg. The transmission electron micrograph (TEM) shown in Figure 1 shows that the average size of the particles was 2.8 nm, with an average of 807 gold atoms/particle, siRNA and 100 molecules of the glucose derivative as represented in Figure 4 and has an approximate MW >160,000.

### f. Checking the presence of RNA in nanoparticles

RNA-Au-Glc nanoparticles, Glc-Au nanoparticles and the residue of washing the RNA-Au-Glc nanoparticles which presumably containing RNA oligonucleotide and glucose derivative were each dissolved in 30µL of water. Aliquots of these solutions (1µL) were mixed with aqueous solution of ethidium bromide (EtBr) (1 µL, 0.1% v/v). Fluorescence was observed under a UV lamp (see Figure 2) *demonstrating* that the so-prepared nanoparticles have incorporated siRNA (figure 2b, tube 1), while the nanoparticles containing only glucose do not show any fluorescence (figure 2b, tube 2).

4 mg of the siRNA/nanogold complex generated from 148 µg siRNA after were dissolved in 1 ml water to obtain a 6±1 µM stock solution in 20 mM tris. Each µl of solution contained the equivalent of 0.078 µg siRNA.

### Cell Plating

1. 24 h before transfection, 6 x 10⁴ cells were pipetted into a 24-well plate and the volume made up to 0.5 ml with appropriate culture medium.
2. The cells were allowed to reach 50-80 % confluency, taking approximately 24 h.
3. The culture medium was removed and replaced with 300 µl fresh medium/well.

### Transfecting cells with siRNA Complex

1. 3.3 µl (or 12.8 µl of the nanogold complex) of the appropriate duplex siRNA stock was dispensed into a 24-well plate corresponding to that with cells.
2. To each well 96.7 µl (or 87.2 µl for the nanogold complex) of the appropriate culture medium was added and mixed well by pipetting up and down 5 times.
3. To each well (other than the nanogold complex wells) 6 µl of RNAiFect was added and mixed well by pipetting up and down 5 times.
4. The solutions were incubated at room temperature for 10-15 minutes to allow complex formation.
5. The cells in 300 µl culture medium were overlayed with the 100 µl of the appropriate transfection complex.
6. The plate gently rocked to mix avoiding swirling.
7. The plate was incubated at 37°C in a CO₂ incubator for 48-72 h.
8. The medium was removed and the cells washed three times with ice cold PBS.
9. The cells were lysed and the protein content of the lysates determined.
10. The proteins were separated by SDS-PAGE followed by Western blotting using a Her2/ErbB2 polyclonal rabbit antibody from Cell Signalling Technology (Cat.# 2242).
11. The blots were treated with an anti-rabbit IgG-HRP conjugate followed by ECL development.

### g. Results

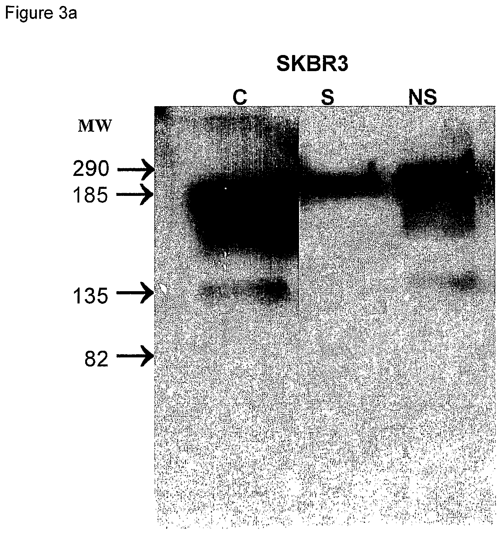

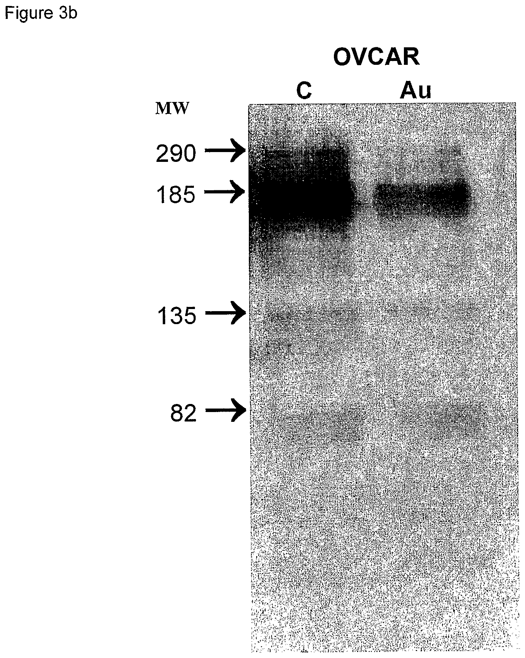

Preliminary observations using 1µg siRNA/well are shown in Figures 3a and 3b. siRNA-gold nanoparticles were added to cells without RNAiFectamine. The SKBR3 cells were slower to reach 80% confluency than the OVCAR cells. The SKBR3 results are from lysates 48h after transfection while the OVCAR results were from lysates 72h after transfection. A schematic of the nanoparticles are shown in Figure 4.

### Non-toxicity of siRNA-Au-Glc nanoparticles to cells

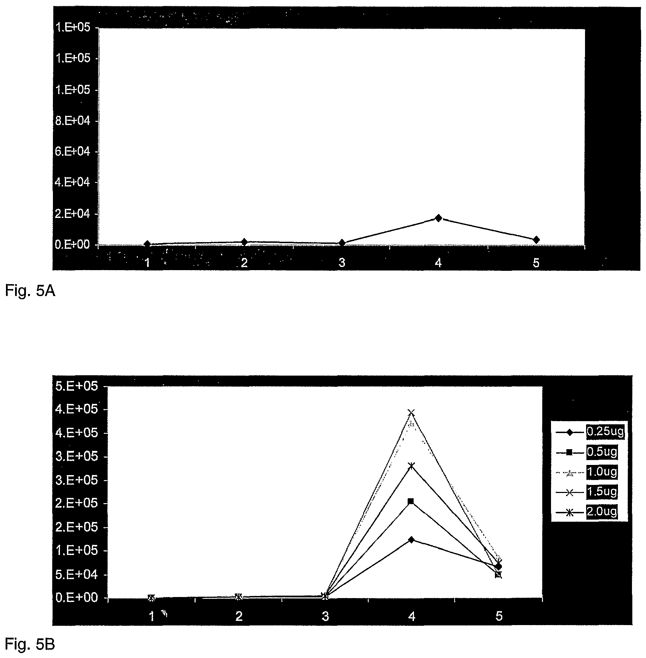

Cells were transfected with siRNA alone and with siRNA conjugated to gold glyconanoparticles. Figure 5 shows that the nanoparticle-conjugated siRNA was effective and had no toxic effects. A dose-dependent effect on cell number was seen indicating that the siRNA nanoparticles increased cell proliferation.

### Entry of siRNA-Au-Glc nanoparticles into cells

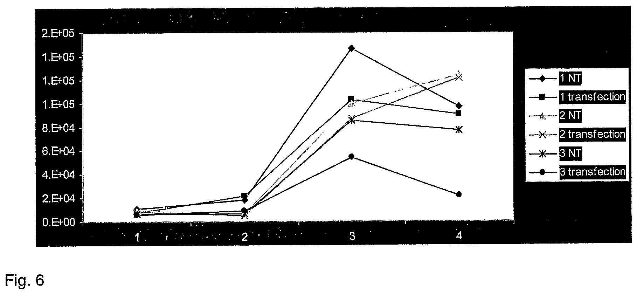

OVCAR cells were transfected with siRNA-nanoparticles with and without the transfection reagent usually required for transfection of cells with siRNA. Figure 6 shows that the transfection reagent was not required for entry of siRNA-nanoparticles into the cells. The results show that the siRNA nanoparticles were effectively delivered into the cells even in the absence of transfection reagent; indeed, delivery appeared tobe more efficient without transfection reagent. The dose-dependency of the effect on cell number indicates a genuine response to the siRNA-nanoparticles.

### References

Pal-Bhadra et al. RNAi-Mediated targeting of Heterochromatin by the RITS complex. Science (2004) vol. 303, 669.
Verdel et al. Heterochromatic silencing and HP1 localization in Drosphila are dependent on the RNAi Machinery. Science (2004) vol. 303, 672.
Elbashir et al. Duplexes of 21-nucleotide RNAs mediate RNA interference in cultured mammalian cells. Nature 2001;411:494-8.
Tuschl. RNA interference and small interfering RNAs. Chem Biochem 2001;2:239-45.
Bargmann et al. The neu oncogene encodes an epidermal growth factor receptor-related protein. (1986) Nature 319, 226-230.
Yarden & Sliwkowski. Untangling the ErbB signalling network. (2001) Nat Rev Mol Cell Biol 2, 127-137.
Berchuck et al. Overexpression of HER-2/neu is associated with poor survival in advanced epithelial ovarian cancer. (1990) Cancer Res 50, 4087-4091.
Mendelsohn & Baselga. The EGF receptor family as targets for cancer therapy. (2000) Oncogene 19, 6550-6565.
Baselga et al. Phase II study of weekly intravenous recombinant humanized anti-p185HER2 monoclonal antibody in patients with HER2/neu over expressing metastatic breast cancer. J Clin Oncol 1996;14:737-44.
Roh et al. Down regulation of HER2/neu expression induces apoptosis in human cancer cells that over-express HER2/neu. (2000) Cancer Res 60, 560-565.
Chang et al. Inhibition of intratracheal lung cancer development by systemic delivery of E1A. (1996) Oncogene 13, 1405-1412.
Sherr & Roberts. CDK inhibitors: positive and negative regulators of G1-phase progression. (1999) Genes Dev 13, 1501-1512.
Miyagishi et al. Comparison of the suppressive effects of antisense oligonucleotides and siRNAs directed against the same targets in mammalian cells. Antisense Nucleic Acid Drug Dev 2003;13:1-7.
Kumar & Yarmand-Bagheri. The role of HER2 in angiogenesis. (2001) Semin Oncol 28, 27-32.
Izumi et al. Tumour biology: herceptin acts as an antiangiogenic cocktail. (2002) Nature 416, 279-280.
Choudhury et al. Small interfering RNA (siRNA) inhibits the expression of the Her2/Neu gene, upregulates HLA Class I and induces apoptosis of Her2/Neu positive tumour cell lines. Int J Cancer 108, 71-77, 2004.
Overbaugh, HTLV sweet-talks its way into cells, Nat. Med (2004) vol. 10, 20.
Check. RNA to the rescue. Nature (2003)vol 425, 10 - 12.
Zamore et al. siRNAs knock down hepatitis. Nature (2003) vol 9, 266-267.
Song et al. RNA interference targeting Fas protects mice from fulminant hepatitis. Nat. Med. (2003)vol. 9, 347 - 351.
Matzke & Matzke. RNAi Extends its Reach. Science (2003) Vol 301, 1060 - 1061.
WO 02/32404
PCT application claiming priority from GB-A-0313259.4.

## Claims

1. A method for detecting and/or imaging mRNA employing nanoparticles which comprise a core including metal and/or semiconductor atoms, wherein the core is covalently linked to a plurality of ligands which comprise:
(i) at least one RNA ligand comprising (a) a siRNA or miRNA molecule between 17 and 30 ribonucleotides in length; and (ii) a ligand comprising a carbohydrate group,
wherein the method comprises contacting the nanoparticles with a sample or cells containing target mRNA under conditions in which the RNA ligands present on the nanoparticles are capable of interacting with target mRNA and detecting the nanoparticle-RNA-mRNA complex,
and wherein the core of the nanoparticle has a mean diameter between 0.5 and 10 nm.

2. The method of claim 1, wherein the step of detecting the complex uses an inherent property of the nanoparticles or by detecting a label associated with the nanoparticle.

3. The method of claim 2, wherein the label is a magnetic label, a quantum dot or a radionuclide

4. The method of any one of the preceding claims, wherein the method is carried out *in vitro* on a sample containing the mRNA.

5. The method of any one of the preceding claims, wherein the method is for detecting mRNA in cells.

6. The method of any one of the preceding claims, wherein the method is a tool for functional genomics.

7. The method of any one of the preceding claims, wherein the nanoparticle is covalently linked to the RNA ligand via a linker group.

8. The method of claim 7, wherein the linker group is a thiol group, an ethylene group or a peptide group.

9. The method of any one of the preceding claims, wherein the ligand is a siRNA ligand and comprises a 3' overhang of 2 ribonucleotides.

10. The method of any one of the preceding claims, wherein a first sense or antisense strand of a RNA molecule is covalently linked to a nanoparticle core via its 5' and/or 3' end.

11. The method of claim 10, wherein a second strand of the RNA molecule which is complementary to the first strand is annealed to the first strand of the RNA molecule, optionally wherein the second RNA strand is covalently linked to a nanoparticle core via its 5' and/or 3' end.

12. The method of claim 11, wherein the first and second strands of the RNA molecule are separately linked to nanoparticle cores and subsequently annealed together.

13. The method of any one of the preceding claims, wherein the core of the nanoparticle comprises gold atoms.

## Patentansprüche

1. Verfahren zum Detektieren und/oder Abbilden von mRNA unter Einsatz von Nanoteilchen, die einen Kern umfassen, der Metall- und/oder Halbleiteratome umfasst, wobei der Kern kovalent an eine Vielzahl von Liganden gebunden ist, die Folgendes umfassen:
(i) zumindest einen RNA-Liganden, der (a) ein siRNA- oder miRNA-Molekül mit einer Länge zwischen 17 und 30 Ribonucleotiden umfasst; und
(ii) einen Liganden, der eine Kohlenhydratgruppe umfasst,
wobei das Verfahren das Kontaktieren der Nanoteilchen mit einer Probe oder Zellen, die Ziel-mRNA enthalten, unter Bedingungen, unter denen die auf den Nanoteilchen vorhandenen RNA-Liganden in der Lage sind, mit Ziel-mRNA wechselzuwirken, und das Detektieren des Nanoteilchen-RNA-mRNA-Komplexes umfasst
und wobei der Kern des Nanoteilchens einen mittleren Durchmesser zwischen 0,5 und 10 nm aufweist.

2. Verfahren nach Anspruch 1, wobei im Schritt des Detektierens des Komplexes eine den Nanoteilchen inhärente Eigenschaft genutzt wird oder durch Detektieren einer dem Nanoteilchen zugeordneten Markierung erfolgt.

3. Verfahren nach Anspruch 2, wobei die Markierung eine magnetische Markierung, ein Quantenpunkt oder ein Radionuclid ist.

4. Verfahren nach einem der vorangegangenen Ansprüche, wobei das Verfahren in vitro an einer die mRNA enthaltenden Probe durchgeführt wird.

5. Verfahren nach einem der vorangegangenen Ansprüche, wobei das Verfahren dem Detektieren von mRNA in Zellen dient.

6. Verfahren nach einem der vorangegangenen Ansprüche, wobei das Verfahren ein Werkzeug für Funktionsgenomik ist.

7. Verfahren nach einem der vorangegangenen Ansprüche, wobei das Nanoteilchen über eine Linkergruppe kovalent an den RNA-Liganden gebunden ist.

8. Verfahren nach Anspruch 7, wobei die Linkergruppe eine Thiolgruppe, eine Ethylengruppe oder eine Peptidgruppe ist.

9. Verfahren nach einem der vorangegangenen Ansprüche, wobei der Ligand ein siRNA-Ligand ist und einen 3'-Überhang von 2 Ribonucleotiden umfasst.

10. Verfahren nach einem der vorangegangenen Ansprüche, wobei ein erster Sense- oder Antisense-Strang eines RNA-Moleküls über sein 5'- und/oder 3'-Ende kovalent an einen Nanoteilchenkern gebunden ist.

11. Verfahren nach Anspruch 10, wobei ein zweiter Strang des RNA-Moleküls, der zum ersten Strang komplementär ist, an den ersten Strang des RNA-Moleküls anelliert wird, wobei gegebenenfalls der zweite RNA-Strang über sein 5'- und/oder 3'-Ende kovalent an einen Nanoteilchenkern gebunden ist.

12. Verfahren nach Anspruch 11, wobei der erste und zweite Strang des RNA-Moleküls separat an Nanoteilchenkerne gebunden und daraufhin aneinander anelliert werden.

13. Verfahren nach einem der vorangegangenen Ansprüche, wobei der Kern des Nanoteilchens Goldatome umfasst.

## Revendications

1. Procédé de détection et/ou d'imagerie d'ARNm employant des nanoparticules qui comprennent un noyau comprenant des atomes métalliques et/ou semi-conducteurs, où le noyau est lié de façon covalente à une pluralité de ligands qui comprennent :
(i) au moins un ligand d'ARN comprenant (a) une molécule d'ARNsi ou d'ARNmi ayant une longueur comprise entre 17 et 30 ribonucléotides ; et
(ii) un ligand comprenant un groupe glucide,
où le procédé comprend la mise en contact des nanoparticules avec un échantillon ou des cellules contenant un ARNm cible dans des conditions dans lesquelles les ligands d'ARN présents sur les nanoparticules sont capables d'interagir avec l'ARNm cible, et la détection du complexe nanoparticule-ARN-ARNm,
et où le noyau de la nanoparticule possède un diamètre moyen compris entre 0,5 et 10 nm.

2. Procédé selon la revendication 1, dans lequel l'étape de détection du complexe utilise une propriété inhérente des nanoparticules ou détecte un marqueur associé à la nanoparticule.

3. Procédé selon la revendication 2, dans lequel le marqueur est un marqueur magnétique, une boîte quantique ou un radionucléide.

4. Procédé selon l'une quelconque des revendications précédentes, où le procédé est réalisé *in vitro* sur un échantillon contenant l'ARNm.

5. Procédé selon l'une quelconque des revendications précédentes, où le procédé est destiné à détecter de l'ARNm dans des cellules.

6. Procédé selon l'une quelconque des revendications précédentes, où le procédé est un outil pour la génomique fonctionnelle.

7. Procédé selon l'une quelconque des revendications précédentes, dans lequel la nanoparticule est liée de façon covalente au ligand d'ARN via un groupe de liaison.

8. Procédé selon la revendication 7, dans lequel le groupe de liaison est un groupe thiol, un groupe éthylène ou un groupe peptidique.

9. Procédé selon l'une quelconque des revendications précédentes, dans lequel le ligand est un ligand d'ARNsi et comprend un porte-à-faux 3' de 2 ribonucléotides.

10. Procédé selon l'une quelconque des revendications précédentes, dans lequel un premier brin sens ou antisens d'une molécule d'ARN est lié de façon covalente à un noyau de nanoparticule via son extrémité 5' et/ou 3'.

11. Procédé selon la revendication 10, dans lequel un second brin de la molécule d'ARN qui est complémentaire au premier brin est annelé au premier brin de la molécule d'ARN, facultativement dans lequel le second brin d'ARN est lié de façon covalente à un noyau de nanoparticule via son extrémité 5' et/ou 3'.

12. Procédé selon la revendication 11, dans lequel le premier et le second brin de la molécule d'ARN sont séparément liés à des noyaux de nanoparticule et sont ensuite annelés l'un à l'autre.

13. Procédé selon l'une quelconque des revendications précédentes, dans lequel le noyau de la nanoparticule comprend des atomes d'or.
